# EUROPEAN PATENT SPECIFICATION

(11) **EP 3 225 217 B1**
(45) Date of publication and mention of the grant of the patent: **12.09.2018**
(21) Application number: 17162444.8
(22) Date of filing: 22.03.2017
(51) Int. Cl.: A61F 2/915

(54) **STENT**
STENT
ENDOPROTHÈSE

(30) Priority: 28.03.2016 JP 2016064174
(43) Date of publication of application: 04.10.2017
(73) Proprietor: Terumo Kabushiki Kaisha, Shibuya-ku Tokyo (JP)
(72) Inventor: IKEUCHI, Ryouta, Fujinomiya-shi, Shizuoka 418-0015 (JP); MUKADA, Miho, Fujinomiya-shi, Shizuoka 418-0015 (JP)
(74) Representative: Casalonga

(56) References cited:
- EP-A2- 1 129 673
- US-A1- 2002 007 212
- US-A1- 2002 116 049

## Description

### [Technical Field]

The present invention relates to a stent.

### [Background Art]

In particular, the invention relates to a stent according to the preamble of claim 1, such as it is e.g known from EP1 129 673 A2.

A stent is a device which maintains a patency state of a blood vessel after being indwelled in, for example, a stenosed portion or a closed portion, which is generated in the blood vessel, in an expanded state. A cylindrical circumference in which gaps are formed is formed in a stent using a linear strut (refer to Patent Literature 1).

### [Citation List]

### [Patent Literature]

[Patent Literature 1] International Publication No. 2007/040250

### [Summary of the Invention]

### [Problem that the Invention is to Solve]

A stent of which the length during expansion is unlikely reduced and which has satisfactory flexibility so as to be able to pass through a biological lumen is required, in order to accurately indwell the stent in a target lesion area.

However, in reality, there is no stent compatible with these points.

Insofar as the terms "invention" and/or "embodiment" are used in the following, and/or features are presented as being optional, this should be interpreted in such a way that the only protection sought is that of the invention as claimed. An object of the present invention is to provide a stent which has satisfactory flexibility so as to be able to pass through a biological lumen and of which the length during expansion is unlikely reduced, and therefore, it is possible to accurately indwell the stent in a target lesion area.

### [Means for Solving the Problem]

The present invention for achieving the above-described purpose is a stent having linear struts forming a cylindrical circumference. Each strut has a wave shape in which a plurality of mountains as convex portions are formed toward both sides of the cylindrical shape in an axial direction. Annular reference bodies which are units repeatedly arranged in the axial direction include at least a first annular portion configured to form the strut in an annular shape, a second annular portion which is configured to form the strut in an annular shape and is adjacent to the first annular portion in the axial direction, and a plurality of first link portions which are disposed in a circumferential direction of the cylindrical shape between the first annular portion and the second annular portion adjacent to each other in the axial direction, and connect the first annular portion to the second annular portion. The stent includes a plurality of second link portions which are disposed in the circumferential direction of the cylindrical shape between the annular reference bodies adjacent to each other in the axial direction, and connect the annular reference bodies to each other. Intervals between the first link portions adjacent to each other in the circumferential direction are equal and intervals between the second link portions adjacent to each other in the circumferential direction are equal. The number of mountains in the first annular portion and the number of mountains in the second annular portion between the first link portions adjacent to each other in the circumferential direction differ from each other. The first link portions are positioned on a first line parallel to the axial direction and the second link portions are positioned on a second line parallel to the axial direction, in a state where the plurality of annular reference bodies are repeatedly arranged in the axial direction.

### [Advantage of the Invention]

According to the stent constituted as described above, the first annular portion in which the strut "densely" exists and the second annular portion in which the strut "sparsely" exists are regularly repeatedly arranged in the axial direction. Furthermore, a first link portion is coaxially positioned (on a first line) and a second link portion is coaxially positioned (on a second line). For this reason, the stent becomes a stent of which an expansion state and an expansion timing become uniform in the axial direction and the length during expansion is unlikely reduced and which has satisfactory flexibility so as to be able to pass through a biological lumen.

### [Brief Description of the Drawings]

[Fig. 1] Fig. 1 is a perspective view showing a balloon catheter on which a stent is mounted.
[Fig. 2] Fig. 2 is a perspective view showing an enlarged distal portion of the balloon catheter shown in Fig. 1.
[Fig. 3] Fig. 3 is a development view, in which a part of the circumference of the stent is cut into a linear shape and is developed along an axial direction, and is a development view before the stent is expanded.
[Fig. 4] Fig. 4 is a partially enlarged view of Fig. 3.
[Fig. 5] Fig. 5 is a development view, in which a part of the circumference of the stent is cut into a linear shape and is developed along the axial direction, and is a development view when the stent is expanded.

### [Best Mode for Carrying Out the Invention]

Hereinafter, an embodiment of the present invention will be described with reference to the accompanying drawings. Note that the following description does not restrict a technical scope disclosed in Claims or the definition of terms. In addition, in some cases, dimensional ratios in the drawings are exaggerated and are different from the actual ratios for the convenience of description. In addition, in the description below, the hand operation side of a medical device will be referred to as a "proximal side", and the side through which the medical device is inserted into the biological lumen will be referred to as a "distal side".

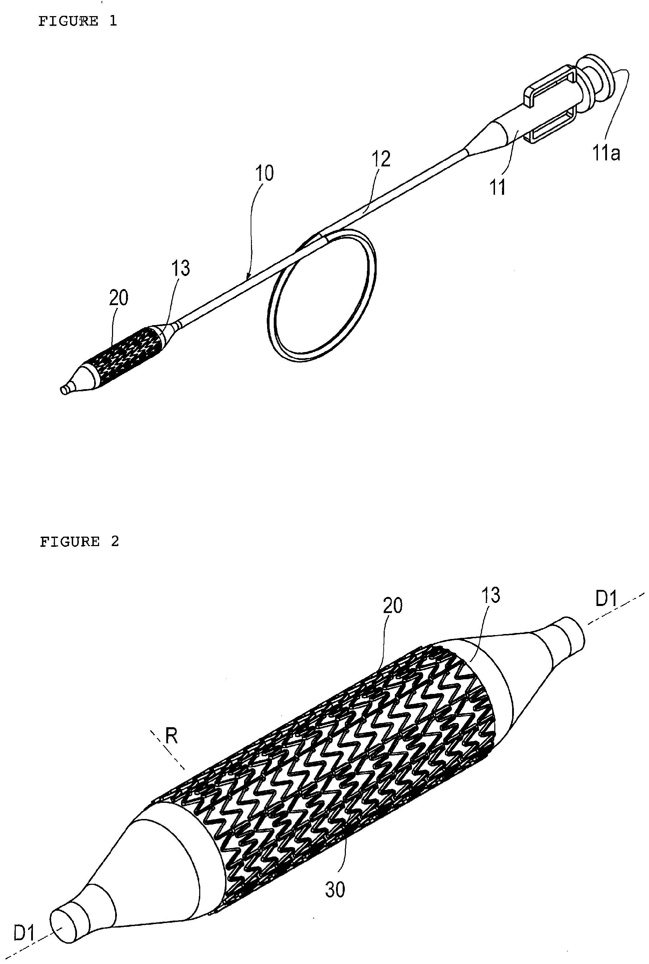

Fig. 1 is a perspective view showing a balloon catheter 10 on which a stent 20 is mounted and Fig. 2 is a perspective view showing an enlarged distal portion of the balloon catheter 10 shown in Fig. 1.

When referring to Fig. 1, the balloon catheter 10 has a hub 11 disposed in a proximal portion, an elongated shaft portion 12, and a dilatable balloon 13 which is disposed in a distal portion of the shaft portion 12. When also referring to Fig. 2, the stent 20 has a cylindrical shape and is mounted on the balloon 13. The stent 20 has a linear strut 30 which forms a cylindrical circumference.

The hub 11 has an interlock port 11a with which an indeflator for supplying a fluid for dilating the balloon 13 is interlock. Examples of the dilation fluid for dilating the balloon 13 include an X-ray contrast agent, a physiological salt solution, and an electrolytic solution, but are not limited thereto.

The shaft portion 12 has a lumen which allows communication between the interlock port 11a of the hub 11 and the inside of the balloon 13.

The balloon 13 is fixed to the circumference of the shaft portion 12 in the vicinity of the distal portion and is disposed in a state of being folded (or a state of being deflated). The inside of the balloon 13 communicates with the interlock port 11a of the hub 11 via the lumen. A dilation fluid which has been made to flow in from the interlock port 11a of the hub 11 flows into the balloon 13. The balloon 13 is dilated due to the dilation fluid which has been introduced.

The constituent material of the balloon 13 preferably has a flexibility, and examples thereof include polymer materials such as polyolefins, a cross-linked body of polyolefins, polyester, polyester elastomer, polyvinyl chloride, polyurethane, polyurethane elastomer, polyphenylene sulfide, polyamide, polyamide elastomer, and fluororesin, silicone rubber, and latex rubber. Polyester is, for example, polyethylene terephthalate. The constituent material of the balloon 13 is not limited to a form of singly using the above-described polymer material, and it is also possible to apply, for example, a film on which the above-described polymer material is appropriately stacked.

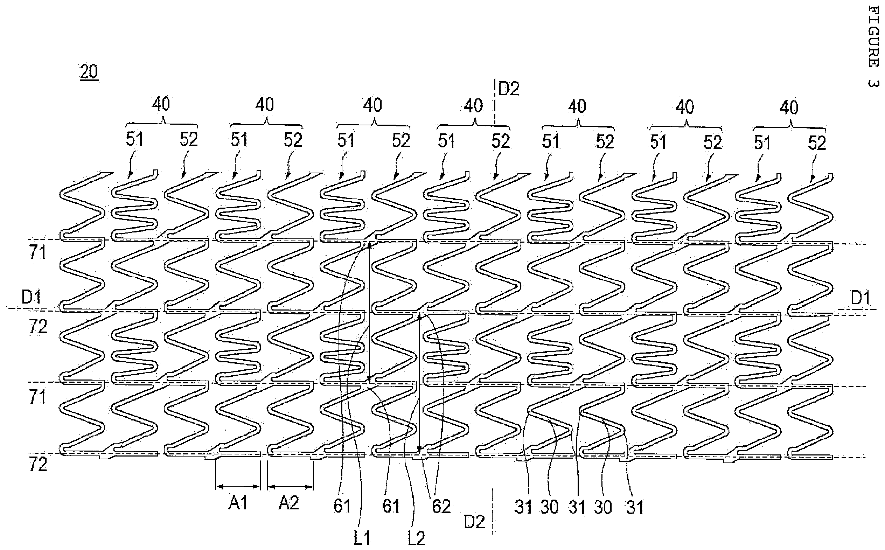

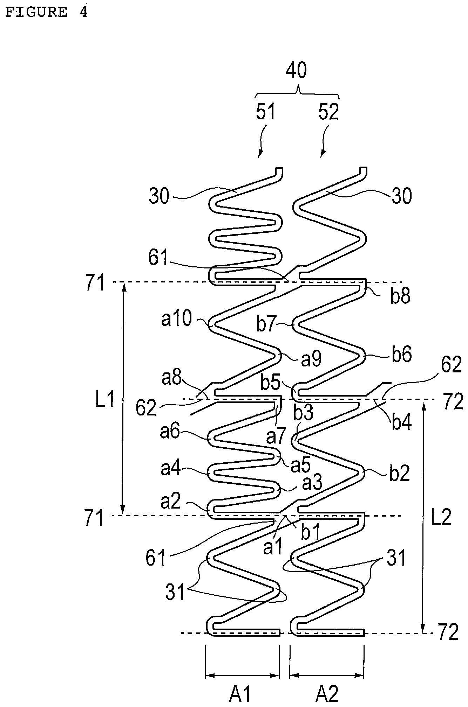

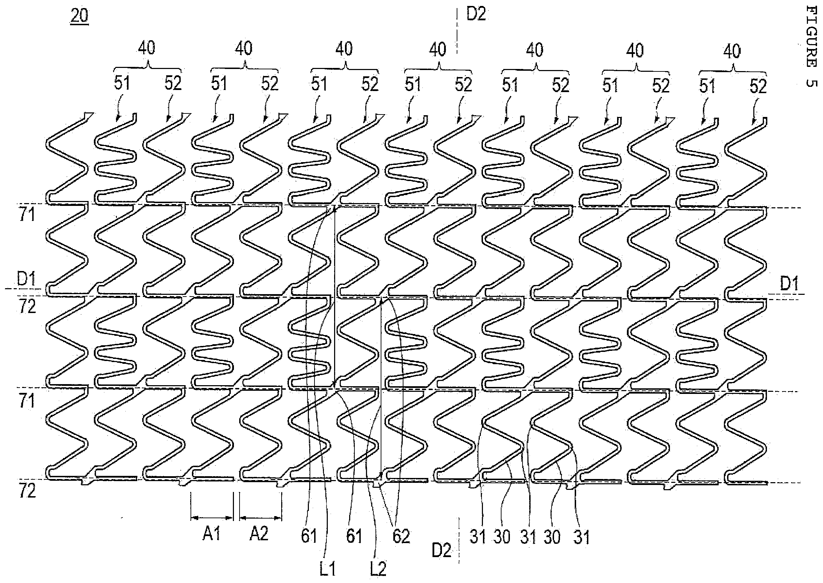

Fig. 3 is a development view, in which a part of the circumference of the stent 20 is cut into a linear shape and is developed along an axial direction, and is a development view before the stent 20 is expanded. Fig. 4 is a partially enlarged view of Fig. 3. Fig. 5 is a development view, in which a part of the circumference of the stent 20 is cut into a linear shape and is developed along the axial direction, and is a development view when the stent 20 is expanded.

When referring to Figs. 3 to 5, the stent 20 of the embodiment has the strut 30 which is a linear component. Note that, in the present specification, the axial direction of the cylindrical shape formed by the strut 30 is simply denoted as an "axial direction D1" (refer to Figs. 2, 3, and 5), the circumferential direction of the cylindrical shape is simply denoted as a "circumferential direction D2" (refer to Figs. 3 and 5), and the radial direction of the cylindrical shape is simply denoted as a "radial direction R" (refer to Fig. 2) .

The strut 30 forms the circumference of the cylindrical shape in which gaps are formed, as shown in Fig. 2. In addition, the strut 30 extends in the circumferential direction D2 while being folded back in a wave shape, and forms an endless tubular shape.

When referring to Figs. 3 to 5, the strut 30 has a wave shape in which a plurality of mountains 31 (or peaks) as convex portions are formed toward both sides (both right and left sides in the drawings) in the axial direction D1.

The stent 20 of the embodiment has annular reference bodies 40 which are units repeatedly arranged in the axial direction D1. In the illustrated example, seven annular reference bodies 40 are arranged in the axial direction D1.

An annular reference body 40 has a first annular portion 51 which is configured to form the strut 30 in an annular shape, and a second annular portion 52 which is configured to form the strut 30 in an annular shape and adjacent to the first annular portion 51 in the axial direction D1. The annular reference body 40 further has a plurality of first link portions 61 (two in the present embodiment) which are disposed in the circumferential direction D2 between the first annular portion 51 and the second annular portion 52 adjacent to each other in the axial direction D1. The first link portion 61 connects the first annular portion 51 and the second annular portion 52 to each other.

The stent 20 has a plurality of second link portions 62 (two in the present embodiment) which are disposed in the circumferential direction D2 between the annular reference bodies 40 adjacent to each other in the axial direction D1. The second link portion 62 connects the annular reference bodies 40 to each other.

The first annular portion 51 and the second annular portion 52 in one annular reference body 40 are connected using the first link portion 61, and the second annular portion 52 and the first annular portion 51 which are adjacent to each other and are included in the adjacent annular reference bodies 40 are connected to each other using the second link portion 62.

An interval (L1) between the first link portions 61 adjacent to each other in the circumferential direction D2 is equal. In addition, an interval (L2) between the second link portions 62 adjacent to each other in the circumferential direction D2 is equal.

The number of mountains 31 in the first annular portion 51 and the number of mountains 31 in the second annular portion 52 between the first link portions 61 adjacent to each other in the circumferential direction D2 differ from each other. As shown in Fig. 4, the number of mountains 31 in the first annular portion 51 is ten (a1 to a10) and the number of mountains 31 in the second annular portion 52 is eight (b1 to b8). Regarding the mountains 31 in the first annular portion 51, mountains 31 (a1, a3, a5, a7, and a9) become convex portions toward a right side in the drawing in the axial direction D1 and mountains 31 (a2, a4, a6, a8, and a10) become convex portions toward a left side in the drawing in the axial direction D1. Regarding the mountains 31 in the second annular portion 52, mountains 31 (b2, b4, b6, and b8) become convex portions toward a right side in the drawing in the axial direction D1 and mountains 31 (b1, b3, b5, and b7) become convex portions toward a left side in the drawing in the axial direction D1. The distance, that is, the amplitude between the mountains 31 (a1, a3, a5, a7, and a9) and the mountains 31 (a2, a4, a6, a8, and a10) in the first annular portion 51 in the axial direction D1 is represented by a sign A1. The distance, that is, the amplitude between the mountains 31 (b2, b4, b6, and b8) and the mountains 31 (b1, b3, b5, and b7) in the second annular portion 52 in the axial direction D1 is represented by a sign A2. The amplitude A1 of the first annular portion 51 is equal to the amplitude A2 of the second annular portion 52.

The number of mountains 31 (ten (a1 to a10)) in the first annular portion 51 is greater than the number of mountains 31 (eight (b1 to b8)) in the second annular portion 52. In addition, the amplitude A1 of the first annular portion 51 is equal to the amplitude A2 of the second annular portion 52. Accordingly, the total length of the strut 30 in the first annular portion 51 is longer than the total length of the strut 30 in the second annular portion 52. In other words, the strut 30 of the first annular portion 51 "densely" exists relative to the strut 30 of the second annular portion 52. In addition, it can be said that the strut 30 of the second annular portion 52 "sparsely" exists relative to the strut 30 of the first annular portion 51. The amount of pinching the balloon 13 using the strut 30 during crimping is larger in the second annular portion 52 in which the strut 30 "sparsely" exists than in the first annular portion 51 in which the strut 30 "densely" exists. Accordingly, when the balloon 13 is dilated, the second annular portion 52 of the stent 20 is easily expanded than the first annular portion 51.

As shown in Figs. 3 and 5, the first link portions 61 are positioned on first lines 71 parallel to the axial direction D1 and the second link portions 62 are positioned on the second lines 72 parallel to the axial direction D1, in a state where the plurality of annular reference bodies 40 are repeatedly arranged in the axial direction D1. Note that it is sufficient for the "first line 71" and the "second line 72" to have substantially linear shape, and it is understood that a small curved portion or a case of partially having a small bent portion is also included.

Since the first link portions 61 are coaxially positioned (on the first lines 71) and the second link portions 62 are coaxially positioned (on the second lines 72), it is possible to secure the collapse strength in the axial direction D1, and therefore, the length variation (shortening) of the stent 20 during expansion in the axial direction D1 becomes almost zero. In addition, since the stent 20 has a high strength in the axial direction D1, it is difficult for the stent 20 to be deformed (deformation) in the axial direction D1 when delivering the stent 20 using the balloon catheter 10.

It is preferable that the first lines 71 on which the first link portions 61 are positioned and the second lines 72 on which the second link portions 62 are positioned are offset to each other in the circumferential direction D2.

When comparing the case with a case where the first lines 71 and the second lines 72 are coaxially positioned together, twisting of the whole stent 20 in the circumferential direction D2 or deflection from the axial direction D1 is easily caused, and therefore, the flexibility of the stent 20 is improved. As a result, it is possible to obtain more satisfactory passing properties of the stent 20 at a position of a curved lesion or the like.

The number of first link portions 61 in one annular reference body 40 and the number of second link portions 62 between the annular reference bodies 40 are equal to each other. Furthermore, the plurality of second link portions 62 preferably connect the annular reference bodies 40 at a central position (in the present embodiment, a position which becomes 90° to the first link portion 61 in the circumferential direction D2) between the first link portions 61 adjacent to each other in the circumferential direction D2.

An interval (L1) between the first link portions 61 adjacent to each other in the circumferential direction D2 and an interval (L2) between the second link portions 62 adjacent to each other in the circumferential direction D2 are equal to each other, and the first line 71 at which the first link portion 61 is positioned and the second line 72 at which the second link portion 62 is positioned are offset while being evenly spaced in the circumferential direction D2. Accordingly, it is possible to uniformly secure the collapse strength of the stent 20 in the axial direction D1 in the circumferential direction D2 and to uniformly secure the flexibility of the stent 20 regardless of the position of the axial direction D1. As a result, it is possible to obtain more satisfactory passing properties of the stent 20 at a position of a curved lesion or the like while uniformly maintaining the high strength in the axial direction D1, in the circumferential direction D2.

The stent 20 functions as an in-vivo indwelling object which holds the lumen at an appropriate size by being indwelled in a stenosed site which is a lesion area by being closely adhered to the inner surface thereof. The stent 20 has a cylindrical shape and is expanded in accordance with the dilation of the balloon 13. The stent 20 is, for example, a drug eluting stent 20 (DES), in which the outer surface is coated with a drug.

The stent 20 is formed of a material having biocompatibility. Examples of the material having biocompatibility include iron, titanium, aluminum, tin, tantalum or tantalum alloy, platinum or platinum alloy, gold or gold alloy, titanium alloy, nickel-titanium alloy, cobalt-based alloy, cobalt-chromium alloy, stainless steel, zinc-tungsten alloy, and niobium alloy.

A drug with which the outer surface of the stent 20 is coated is a mixture of a biologically/physiologically active substance and a biodegradable polymer. Note that the area to be coated with the drug is not limited to the outer surface of the stent 20, and may be the inner surface of the stent 20, or both of the outer surface and the inner surface.

A method for manufacturing the stent 20 is not particularly limited, but examples thereof include a method for cutting a stent from a tube formed of the above-described material using laser or the like, a method performed through injection molding, and a method for performing stacking (lamination molding) using a 3D printer or the like. The method performed through injection molding or the method for performing stacking using a 3D printer or the like is preferable from the viewpoint of precisely finishing a complicated cross-sectional shape. Furthermore, the method performed through injection molding is particularly preferable from the viewpoints of manufacturing a stent at low cost and finishing the surface in a smooth state.

An operation of the present embodiment will be described.

The distal portion of the balloon catheter 10 is positioned at a lesion area and a dilation fluid is introduced into the balloon 13 to dilate the balloon 13. The stent 20 is expanded in the radial direction R in accordance with the dilation of the balloon 13.

In the stent 20, the first annular portion 51 in which the strut 30 "densely" exists and the second annular portion 52 in which the strut 30 "sparsely" exists are regularly repeatedly arranged in the axial direction D1 (refer to Fig. 3). The amount of pinching the balloon 13 using the strut 30 during crimping is larger in the second annular portion 52 in which the strut 30 "sparsely" exists than in the first annular portion 51 in which the strut 30 "densely" exists. Accordingly, when the balloon 13 is dilated, the second annular portion 52 of the stent 20 is easily expanded than the first annular portion 51.

In a case where the amount of pinching the balloon 13 using the strut is made to be uniform in the axial direction D1, both end portions of the balloon 13 in the axial direction D1 are easily dilated. Therefore, the stent is expanded such that both end portions of the stent in the axial direction D1 is first bulged, and then, a central portion is bulged in accordance with the bulging of the both end portions. In contrast, as the present embodiment, in a case where the amount of pinching the balloon 13 using the strut 30 is changed at regular sparseness and denseness in the axial direction D1, the stent 20 is expanded so as to be almost uniformly bulged in the axial direction D1, compared to a case where the pinching amount is made to be uniform. As a result, the expansion state and the expansion timing of the stent 20 becomes uniform in the axial direction D1, and therefore, it is possible to suppress deviation of the stent 20 in the axial direction D1 during the expansion of the stent 20.

After the stent 20 is expanded, the first link portions 61 are coaxially positioned (on the first lines 71) and the second link portions 62 are coaxially positioned (on the second lines 72). For this reason, it is possible to secure the collapse strength in the axial direction D1, and therefore, the length variation (shortening) of the stent 20 during expansion in the axial direction D1 becomes almost zero. In addition, since the stent 20 has a high strength in the axial direction D1, it is difficult for the stent 20 to be deformed (deformation) in the axial direction D1 when delivering the stent 20 using the balloon catheter 10.

In the stent 20, the first annular portion 51 in which the strut 30 "densely" exists and the second annular portion 52 in which the strut 30 "sparsely" exists are regularly repeatedly arranged in the axial direction D1. In other words, since the first annular portion 51 which is relatively rigid and the second annular portion 52 which is relatively flexible are regularly repeatedly arranged in the axial direction D1, passing properties of the stent 20 at a position of a curved lesion or the like become satisfactory. The shrinkage (recoil) in the circumferential direction D2 in the stent 20 during expansion is also reduced due to the first annular portion 51 which is relatively rigid.

In this manner, in the stent 20 of the present embodiment, the variation in the length of the stent during expansion becomes small and the stent has satisfactory flexibility so as to be able to pass through a biological lumen, and therefore, it is possible to accurately indwell the stent in a target lesion area.

An evaluation comparison test was performed on a test product of the stent 20 having the annular reference bodies 40 of which the number of mountains 31 of the first annular portion 51 is ten and the number of mountains 31 of the second annular portion 52 is eight, and an existing product of which the number of mountains 31 of all annular portions is eight. In the test product, the flexibility is excellent by about 10% to 12% due to two-point flexure compared to the existing product, the shortening becomes almost zero, and recoil is also slightly reduced.

In this manner, it is possible to check an effect obtained by the present embodiment in which the stent 20 is uniformly expanded by regularly making variation in the amount of pinching the balloon 13 during crimping using the sparseness and denseness of the stent, and therefore, an improvement in the variation (shortening or recoil) in physical properties of the stent 20 after the expansion can be seen compared to the existing product.

As described above, according to the stent 20 of the present embodiment, the number of mountains 31 in the first annular portion 51 and the number of mountains 31 in the second annular portion 52 are different from each other between the first link portions 61 adjacent to each other in the circumferential direction D2. The first link portions 61 are positioned on the first lines 71 parallel to the axial direction D1 and the second link portions 62 are positioned on the second lines 72 parallel to the axial direction D1, in a state where the plurality of annular reference bodies 40 are repeatedly arranged in the axial direction D1.

According to such a configuration, it is possible to provide the stent 20 which has satisfactory flexibility so as to be able to pass through a biological lumen and in which an expansion state and an expansion timing of the stent 20 becomes uniform in the axial direction D1 and the variation of the stent during expansion is small, and therefore, it is possible to accurately indwell the stent in a target lesion area.

The first lines 71 on which the first link portions 61 are positioned and the second lines 72 on which the second link portions 62 are positioned are offset to each other in the circumferential direction D2.

According to such a configuration, when comparing the case with a case where the first lines 71 and the second lines 72 are coaxially positioned, twisting of the whole stent 20 in the circumferential direction D2 or deflection from the axial direction D1 is easily caused, and therefore, the flexibility of the stent 20 is improved. As a result, passing properties of the stent 20 at a position of a curved lesion or the like becomes more satisfactory.

The number of first link portions 61 in one annular reference body 40 and the number of second link portions 62 between the annular reference bodies 40 are equal to each other. Furthermore, the plurality of second link portions 62 connect the annular reference bodies 40 at a central position between the first link portions 61 adjacent to each other in the circumferential direction D2.

According to such a configuration, it is possible to uniformly secure the collapse strength of the stent 20 in the axial direction D1 in the circumferential direction D2 and to uniformly secure the flexibility of the stent 20 regardless of the position of the axial direction D1. As a result, the passing properties of the stent 20 at a position of a curved lesion or the like becomes more satisfactory while uniformly maintaining the high strength in the axial direction D1, in the circumferential direction D2.

The present invention can be appropriately modified without being limited to the above-described embodiment.

An example of a combination of the number of mountains 31 being ten and eight in the first annular portion 51 and the second annular portion 52 included in the annular reference body 40 has been shown. However, the present invention is not limited to this case. For example, the combination of number of mountains 31 may be set to ten and six. In addition, it is also possible to set a combination of the number of mountains to be a multiple of the number of first link portions 61. The number of mountains 31 is preferably set to a multiple of the number of first link portions 61 in terms of obtaining excellent expansion uniformity.

The embodiment including two annular portions of the first annular portion 51 and the second annular portion 52 in the annular reference body 40 has been shown. However, the present invention is not limited to this case. For example, the present invention may include three or more annular portions and the number of mountains 31 in each of the annular portions may be different from the other. When combining the number of mountains 31, it is possible to appropriately select an arrangement way in a descending order such as ten-nine-eight, an arrangement way in an ascending order such as eight-nine-ten, an arrangement way in which the center is reduced, for example, ten-eight-ten, an arrangement way in which the center is increased, for example, eight-ten-eight, and the like as long as it is possible to arrange the strut 30 by regularly repeating the sparseness and denseness of the strut in the axial direction D1. The case where the amplitude A1 of the first annular portion 51 and the amplitude A2 of the second annular portion 52 are equal to each other has been shown, but may be different from each other.

### [Description of Reference Numerals and Signs]

10 balloon catheter,
11 hub,
12 shaft portion,
13 balloon,
20 stent,
30 strut,
31 mountain,
40 annular reference body,
51 first annular portion,
52 second annular portion,
61 first link portion,
62 second link portion,
71 first line,
72 second line,
D1 axial direction of cylindrical shape,
D2 circumferential direction of cylindrical shape,
L1 interval between first link portions adjacent to each other in circumferential direction,
L2 interval between second link portions adjacent to each other in circumferential direction.

## Claims

1. A stent (20) comprising:
linear struts (30) forming a cylindrical circumference,
wherein each strut (30) has a wave shape in which a plurality of mountains (31) as convex portions are formed toward both sides of the cylindrical shape in an axial direction (D1),
wherein annular reference bodies (40) which are units repeatedly arranged in the axial direction (D1) include at least a first annular portion (51) configured to form the strut (30) in an annular shape,
a second annular portion (52) which is configured to form the strut (30) in an annular shape and is adjacent to the first annular portion (51) in the axial direction (D1), and
a plurality of first link portions (61) which are disposed in a circumferential direction (D2) of the cylindrical shape between the first annular portion (51) and the second annular portion (52) adjacent to each other in the axial direction, and connect the first annular portion (51) to the second annular portion,
wherein the stent (20) includes a plurality of second link portions (62) which are disposed in the circumferential direction (D2) of the cylindrical shape between the annular reference bodies (40) adjacent to each other in the axial direction (D1), and connect the annular reference bodies (40) to each other,
wherein intervals (L1) between the first link portions (61) adjacent to each other in the circumferential direction (D2) are equal,
wherein intervals (L2) between the second link portions (62) adjacent to each other in the circumferential direction (D2) are equal,
wherein the number of mountains (31) in the first annular portion (51) and the number of mountains (31) in the second annular portion (52) between the first link portions (61) adjacent to each other in the circumferential direction (D2) differ from each other, and
**characterized in that** the first link portions (61) are positioned on a first line (71) parallel to the axial direction (D1) and the second link portions (62) are positioned on a second line (72) parallel to the axial direction (D1), in a state where the plurality of annular reference bodies (40) are repeatedly arranged in the axial direction (D1).

2. The stent (20) according to claim 1,
wherein the first line on which the first link portions (61) are positioned and the second line on which the second link portions (62) are positioned are formed to be offset in the circumferential direction (D2).

3. The stent (20) according to claim 1 or 2,
wherein the number of the first link portions (61) in one of the annular reference bodies (40) and the number of the second link portions (62) between the annular reference bodies (40) are equal, and
wherein the plurality of second link portions (62) connect the annular reference bodies (40) at a central position between the first link portions (61) adjacent to each other in the circumferential direction (D2).

## Patentansprüche

1. Stent (20), umfassend:
lineare Streben (30), die einen zylindrischen Umfang bilden,
wobei jede Strebe (30) eine Wellenform besitzt, in der eine Vielzahl von Bergen (31) als konvexe Abschnitte in einer axialen Richtung (D1) zu beiden Seiten der zylindrischen Form hin ausgebildet sind,
wobei ringförmige Referenzkörper (40), die in der axialen Richtung (D1) wiederholt angeordnete Einheiten sind, mindestens einen ersten ringförmigen Abschnitt (51) aufweisen, der dazu ausgelegt ist, die Strebe (30) in einer ringförmigen Gestalt auszubilden,
einen zweiten ringförmigen Abschnitt (52), der dazu ausgelegt ist, die Strebe (30) in einer ringförmigen Gestalt auszubilden und in der axialen Richtung (D1) an den ersten ringförmigen Abschnitt (51) angrenzt, und
eine Vielzahl von ersten Verbindungsabschnitten (61), die in einer Umfangsrichtung (D2) der zylindrischen Form zwischen dem ersten ringförmigen Abschnitt (51) und dem zweiten ringförmigen Abschnitt (52) in der axialen Richtung aneinander angrenzend angeordnet sind und den ersten ringförmigen Abschnitt (51) mit dem zweiten ringförmigen Abschnitt verbinden,
wobei der Stent (20) eine Vielzahl von zweiten Verbindungsabschnitten (62) aufweist, die in der Umfangsrichtung (D2) der zylindrischen Form zwischen den ringförmigen Referenzkörpern (40) in der axialen Richtung (D1) aneinander angrenzend angeordnet sind und die ringförmigen Referenzkörper (40) miteinander verbinden,
wobei Abstände (L1) zwischen den in der Umfangsrichtung (D2) aneinander angrenzenden ersten Verbindungsabschnitten (61) gleich sind,
wobei Abstände (L2) zwischen den in der Umfangsrichtung (D2) aneinander angrenzenden zweiten Verbindungsabschnitten (62) gleich sind,
wobei die Zahl von Bergen (31) in dem ersten ringförmigen Abschnitt (51) und die Zahl von Bergen (31) in dem zweiten ringförmigen Abschnitt (52) zwischen den in der Umfangsrichtung (D2) aneinander angrenzenden ersten Verbindungsabschnitten (61) voneinander verschieden sind, und
**dadurch gekennzeichnet, dass** in einem Zustand, wo die Vielzahl von ringförmigen Referenzkörpern (40) in der axialen Richtung (D1) wiederholt angeordnet sind, die ersten Verbindungsabschnitte (61) auf einer ersten Linie (71) parallel zu der axialen Richtung (D1) positioniert sind und die zweiten Verbindungsabschnitte (62) auf einer zweiten Linie (72) parallel zu der axialen Richtung (D1) positioniert sind.

2. Stent (20) nach Anspruch 1,
wobei die erste Linie, auf der die ersten Verbindungsabschnitte (61) positioniert sind, und die zweite Linie, auf der die zweiten Verbindungsabschnitte (62) positioniert sind, in der Umfangsrichtung (D2) versetzt ausgebildet sind.

3. Stent (20) nach Anspruch 1 oder 2,
wobei die Zahl der ersten Verbindungsabschnitte (61) in einem der ringförmigen Referenzkörper (40) und die Zahl der zweiten Verbindungsabschnitte (62) zwischen den ringförmigen Referenzkörpern (40) gleich sind, und
wobei die Vielzahl von zweiten Verbindungsabschnitten (62) die ringförmigen Referenzkörper (40) an einer zentralen Position zwischen den in der Umfangsrichtung (D2) aneinander angrenzenden ersten Verbindungsabschnitten (61) verbinden.

## Revendications

1. Endoprothèse (20), comprenant :
des entretoises linéaires (30) formant une circonférence cylindrique,
dans laquelle chaque entretoise (30) a une forme ondulée où une pluralité de montagnes (31) sous forme de parties convexes sont formées vers les deux côtés de la forme cylindrique dans une direction axiale (D1),
où des corps de référence annulaires (40) qui sont des unités disposées de façon répétée dans la direction axiale (D1) comprennent au moins une première partie annulaire (51) adaptée pour former l'entretoise (30) dans une forme annulaire,
une seconde partie annulaire (52) qui est adaptée pour former l'entretoise (30) dans une forme annulaire et est adjacente à la première partie annulaire (51) dans la direction axiale (D1), et
une pluralité de premières parties de liaison (61) qui sont disposées dans une direction circonférentielle (D2) de la forme cylindrique entre la première partie annulaire (51) et la seconde partie annulaire (52) adjacentes l'une à l'autre dans la direction axiale, et relient la première partie annulaire (51) à la seconde partie annulaire,
où l'endoprothèse (20) comprend une pluralité de secondes parties de liaison (62) qui sont disposées dans la direction circonférentielle (D2) de la forme cylindrique entre les corps de référence annulaires (40) adjacents les uns aux autres dans la direction axiale (D1), et relient les corps de référence annulaires (40) les uns aux autres,
où des intervalles (L1) entre les premières parties de liaison (61) adjacentes les unes aux autres dans la direction circonférentielle (D2) sont égaux,
où des intervalles (L2) entre les secondes parties de liaison (62) adjacentes les unes aux autres dans la direction circonférentielle (D2) sont égaux,
où le nombre de montagnes (31) dans la première partie annulaire (51) et le nombre de montagnes (31) dans la seconde partie annulaire (52) entre les premières parties de liaison (61) adjacentes les unes aux autres dans la direction circonférentielle (D2) diffère l'un de l'autre, et
**caractérisée en ce que** les premières parties de liaison (61) sont positionnées sur une première ligne (71) parallèle à la direction axiale (D1) et les secondes parties de liaison (62) sont positionnées sur une seconde ligne (72) parallèle à la direction axiale (D1), dans un état où la pluralité de corps de référence annulaires (40) sont disposés de façon répétée dans la direction axiale (D1).

2. Endoprothèse (20) selon la revendication 1,
dans laquelle la première ligne sur laquelle sont positionnées les premières parties de liaison (61) et la seconde ligne sur laquelle sont positionnées les secondes parties de liaison (62) sont formées de manière à être décalées dans la direction circonférentielle (D2).

3. Endoprothèse (20) selon la revendication 1 ou 2,
dans laquelle le nombre des premières parties de liaison (61) dans l'un des corps de référence annulaires (40) et le nombre des secondes parties de liaison (62) entre des corps de référence annulaires (40) sont égaux, et
dans laquelle la pluralité des secondes parties de liaison (62) relient les corps de référence annulaires (40) au niveau d'une position centrale entre les premières parties de liaison (61) adjacentes les unes aux autres dans la direction circonférentielle (D2).
